# EUROPEAN PATENT APPLICATION

(11) **EP 4 198 120 A1**
(43) Date of publication of application: **21.06.2023**
(21) Application number: 20949163.8
(22) Date of filing: 14.08.2020
(51) Int. Cl.: C12N 5/0783, C12N 5/0786, C12N 5/0784, A61K 39/00, A61K 35/17, A61K 35/15, A61P 35/00, A61P 31/18, A61P 1/16, A61P 31/14, A61P 31/20, A61P 31/12, A61P 39/06

(54) **METHOD FOR PREPARING UNIVERSAL IMMUNE CELLS AND USE THEREOF**

(71) Applicant: Shanghai Xinghua Bio Pharmaceutical Science & Technology Co., Ltd, Shanghai 200231 (CN)
(72) Inventor: LIU, Hua, Shanghai 201199 (CN)
(74) Representative: AtlantIP International
(86) International application number: PCT/CN2020/109310
(87) International publication number: WO 2022/032665

(57) **Abstract**

Provided are a simple and convenient method for preparing universal immune cells and the use thereof. The method comprises placing allogeneic immune cells and specific cell mitogens, cytokines, and immunologic adjuvants in a liquid cell culture medium to be co-cultured in a culture container, so as to obtain a universal immune cell culture with a high immunocompetence. Many clinical long-term practical applications have proven that the universal immune cells are safe and reliable, durable and effective, and have no rejection reaction. Therefore, the universal immune cells can be used as maternal cells of other types of universal immune cell preparations, such as CAR-T and TCR-T, and can be used in fields including adoptive cellular immunotherapy, etc.

## Description

### BACKGROUND OF THE INVENTION

### 1. Field of the Invention

The invention relates to the field of biomedicine, and more particularly, to a method for preparing universal immune cells and a use thereof.

### 2. Description of the Related Art

In recent decades, significant progress has been made in the field of tumor treatment. In particular, a cell-based therapy targeting tumor-associated antigens (TAAs) has been developing rapidly. T cells are engineered to have the ability to attack tumor cells by generating CAR constructs consisting of genes encoding scFv, a co-stimulatory domain (CD28 or TNFRSF9), and CD247 signal transduction domain for T cell proliferation and activation. In principle, CAR-T cells are activated by recognizing TAA by scFv on the T cell surface, and then signal transduction domains inside cells connected by scFv are subsequently activated to induce downstream signal transduction pathways involved in T cell proliferation, activation, and production of cytokines.

Currently, CAR-T cell therapy has presented its therapeutic efficacy in treating various diseases, including hematological malignancies, solid tumors, autoimmune diseases, and allergic diseases such as asthma. Furthermore, antigen-specific T regulatory cells (Tregs) and gene-edited T cells appear to be beneficial in controlling inflammation in allergic asthma.

The CAR T therapy is different from traditional drugs. It is mainly an autologous T cell therapy that integrates CAR genes. This therapy has some limitations, that is, a process of haemospasia, isolation, activation, transfection, amplification, preparations-making, release, cryopreservation, transportation and administration must be done quickly. Thus, the CART T cell therapy is a rather expensive and personalized process. It requires expensive measures to collect patient's cells, engineer the collected cells, and reinfuse the cells into the patient's body at each stage, and proper quality control is needed. In addition, traditional chimeric antigen receptors (CARs) have a fixed design, and one type of CAR T cell can only target one antigenic epitope. This rigid design limits clinical applications and leads to extremely high manufacturing costs.

Two CAR-T cell therapies (Yescarta and Kymriah) were approved by the FDA in 2017. The CAR-T cell therapies have become one of the most promising therapies in treating some types of cancers. This success provides an opportunity to optimize the production of CAR T cells, making treatment more accessible to patients. However, two currently FDA-approved T cell products with chimeric antigen receptors (CARs) are derived from autologous T cells. These CART cells approved for clinical use must be generated on a custom-made basis. This autologous T cell production platform remains an important limiting factor for large-scale clinical applications due to the costly and lengthy production process. There is also an inherent risk of production failure. A personalized, custom-made autologous CAR T cell production process also has an effect on the broad application of all types of cancers.

In order to achieve a more comprehensive implementation of advanced cell therapies and to keep costs down, it would be advantageous to make allogeneic "universal" cell therapy products stored in a cell bank in a similar manner to biopharmaceutical drug products and to provide the products upon request. Therefore, the universal allogeneic T cells can be used to generate universal CAR T cells, which can be used as "off-the-shelf" ready-to-use biotherapeutic agents for large-scale clinical applications. As a result, the costs and time required for traditional CAR T cell therapies will be greatly reduced. At present, research and development of the universal CAR-T mainly includes types and preparations of immune cells, and carriers and structures of CAR-T. The main technical routes comprise the elimination of the expression of endogenous αβT cell receptors (TCRs) to prevent graft-versus-host reaction; gene-editing technology; other types of immune cells; and iPSC technology.

When considering allogeneic CAR T cell infusion, host-versus-graft and graft-versus-host rejection must be avoided to prevent rejection of adoptively transferred cells, host tissue damage, and elicitation of significant antitumor outcomes. One modification method currently employed involves knocking out genes that prevent allotherapy, such as endogenous T-cell receptors, while introducing CAR. In doing so, TCRαβ receptors are not available on the cell surface to prevent their alloreactivity. This technique obviously further increases the complexity of the preparation operation, and there is also the hidden danger of knocking out the TCRαβ receptors incompletely.

Since multiple gene targets can be edited simultaneously, some clinical trials have adopted a CRISPR/Cas9 system for CAR-T cell improvement. The combination of these powerful technologies is expected to provide a new generation of CAR-T cell-based immunotherapy for clinical applications, that is, to knock out endogenous TCR through the gene-editing technology, and improve the anti-tumor activity and safety of T cells. However, this method will further increase the complexity of the preparation operation and also increase the production cost.

Natural killer (NK) cells can be xenografted and have the potential to become off-the-shelf products, making CAR-NK cell therapy a universal product. These products may be safer than the CAR-T cell therapy. Scientists report a novel PCa treatment based on NK-92 cell engineering, wherein CAR recognizes human prostate-specific membrane antigen (PSMA), which is overexpressed in prostate tumor cells. After CAR transduction, NK-92/CAR cells acquire a high and specific lytic activity against PSMA-expressing prostate cancer cells *in vitro,* and also degranulate and produce high levels of IFN-γ in response to antigen recognition. However, this technique requires lethal irradiation of effectors, which is a safety measure required for a clinical application of targeted NK-92 cells, which can completely eliminate replication without affecting phenotype and short-term functions.

Other researchers have explored a technical route of developing off-the-shelf cell therapies based on induced pluripotent stem cell (iPSC)-derived natural killer (NK) cells. They proposed strategies to engineer iPSC-derived NK (iPSC-NK) cells to enhance immune cell functions, prolong persistence, and promote cell homing. But so far, there is no report of iPSC-derived CTL (iPSC-CTL).

The antigen-specific immune cell drugs (CN101626781B, CN102847143B, CN102847144B) previously applied by the inventor have been used in the clinical applications for nearly ten years, and have treated nearly 800 tumor patients, ranging in age from 18 months to 93 years. Among these patients, the most frequent use was more than 500 times, and the longest duration was ten years. The total number of peripheral blood leukocytes is in a range of 0.9 - 30.0×10⁹/L. It has been proved by clinical multi-center applications that they are safer, and does not cause graft-versus-host rejection (GVHD) and autoimmune diseases, while about 50-60% of patients treated with traditional DLI develop GVHD.

Therefore, the applicant intends to design a method for preparing universal allogeneic T cells based on previous patents, and to provide maternal(backbone) cells of other types of universal immune cell preparations, such as CAR-T, TCR-T or other immune cells, for use in the fields of adoptive cellular immunotherapy and the like, so as to improve the quality of the immune cells, reduce the costs of the cell therapy and increase its safety.

### SUMMARY OF THE INVENTION

An object of the present invention is to overcome defects in the prior art and to provide a simple and convenient method for preparing universal immune cells and the use thereof. The prepared universal immune cells are safe, reliable, durable and effective, and have no graft-versus-host rejection reaction.

In order to solve the above-mentioned technical problems, the present invention adopts the following technical solutions:
In a first aspect, the present invention provides a method for preparing universal immune cells, comprising the steps of:
Step 1, placing allogeneic immune cells (e.g., mononuclear cells), cell mitogens, cytokines and immunologic adjuvants in a liquid cell culture medium to be co-cultured in a culture container, so that universal immune cell culture is obtained;
Step 2, isolating a cell population from the culture obtained in Step 1;

wherein the cytokines are derived from any one or more of lymphokines, monokines, cytokines activating inflammation and cytokines stimulating hematopoiesis produced by lymphocytes, monocytes and other cells.

Furthermore, the cytokines are derived from any one or more of interleukins, interferons, tumor necrosis factors, colony-stimulating factors, chemotactic cytokines and transforming growth factors.

Furthermore, the cytokines are one or more of interleukin (IL)-2, IL-6, IL-15 and interferons.

Furthermore, the concentration of the cytokines is in a range of 200,000 units/L to 5,000,000 units/L.

Furthermore, the concentration of the cytokines is in a range of 500,000 units/L to 2,000,000 units/L.

Furthermore, the mitogens are selected from any one or more of concanavalin, phytohaemagglutinin, pokeweed, lipopolysaccharide, and dextran.

Furthermore, the concentration of the mitogens is in a range of 100,000 units/L to 10,000,000 units/L.

Furthermore, the concentration of the mitogens is in a range of 0.1 mg/L to 10 mg/L.

Furthermore, the immunologic adjuvants are selected from any one or more of biological adjuvants, inorganic adjuvants, organic adjuvants, synthetic adjuvants, oil-based adjuvants, and Freund's adjuvants, and the concentration of the immunologic adjuvants is in a range of 0.01 mg/L to 1 mg/L.

Furthermore, the immune cells are derived from peripheral blood or umbilical cord blood.

Furthermore, the immune cells may be allogeneic cells or autologous cells.

Furthermore, the concentration of the immune cells is in a range of 1×10³ cells/ml to 1×10¹¹ cells/ml.

Furthermore, the cell population comprises cytotoxic T lymphocytes (CTL), tumor-infiltrating lymphocytes (TIL), cytokine-induced killer (CIK) cells, lymphokine-activated killer (LAK) cells, natural killer (NK) cells, tumor-associated macrophages (TAM), activated killer monocytes (AKM), and dendritic cells (DC).

Furthermore, the cell population with immunocompetence is lymphocytes, such as CTL, NK, CIK and B cells, most preferably the cytotoxic T lymphocytes.

Furthermore, the cells are co-cultured for a period of 3-180 days in Step 1.

Furthermore, the culture container is a three-dimensional cell culture container with a large volume and a high density.

Furthermore, the method further comprises a cloning step: using the culture obtained in Step 1 or the cell population with immunocompetence obtained in Step 2 as raw material, and performing a cloning process in a liquid cell culture medium to obtain cell lines with immunocompetence. The cloning process is performed by intermittent cyclic stimulation or continuous stimulation.

In a second aspect, the present invention provides universal immune cells prepared by the above-mentioned method. The cells comprise one or more of cytotoxic T lymphocytes (CTL), tumor-infiltrating lymphocytes (TIL), cytokine-activated killer (CLK) cells, lymphokine-activated killer (LAK) cells, natural killer (NK) cells, tumor-associated macrophages (TAM), activated killer monocytes (AKM), and dendritic cells (DC).

In a third aspect, the present invention provides a use of the universal immune cell in the second aspect for the preparation of an immune cell preparation, wherein maternal(backbone) cells of the immune cell preparation are the universal immune cells.

Furthermore, the immune cell preparation comprises CAR-T cells or TCR-T cells.

In a fourth aspect, the present invention provides an immune cell preparation prepared by using the universal immune cells in the second aspect as the maternal(backbone) cells.

In a fifth aspect, the present invention provides a biological drug for adoptive cellular immunotherapy, comprising a container and the immune cell preparation in the fourth aspect, placed in the container.

Furthermore, the biological drug further comprises physiological saline, albumin and other stabilizers.

In a sixth aspect, the present invention provides a use of the immune cell preparation in the fourth aspect for the preparation of drugs for the treatment and prevention of diseases related to abnormalities in the quantity and functions of lymphocytes.

Furthermore, routes of administration for the drugs comprise intravenous injection, intrathoracic injection, intraperitoneal injection, intraspinal injection, intradermal injection, subcutaneous injection or intratumoral injection.

By adopting the above-mentioned technical solutions, the present invention has the following beneficial effects:
The method for preparing universal immune cells in the present invention is simple, convenient and easy to operate, the allogeneic immune cells are adopted, the quality of immune cell preparations can be improved, the cost of cell therapy is reduced, and the safety of the cell therapy is improved. In addition, a large number of clinical long-term practical applications prove that the universal immune cells are safe, reliable, durable and effective, and have no rejection reaction. Therefore, the universal immune cells can be used as maternal(backbone) cells of other types of universal immune cell preparations, such as CAR-T, TCR-T or other immune cells, and can be used in the fields of adoptive cellular immunotherapy and the like. According to the invention, the problems of high cost, poor safety, low efficiency and the like of existing cellular immunotherapy are solved, and a new way is provided for the application and popularization of the cellular immunotherapy.

In addition, according to the present invention, the method for preparing drugs changes the way traditional vaccines work. The traditional vaccines are made of pathogenic organisms or antigenic substances thereof. Once the vaccines are delivered to a human body, the immune system will produce protective substances (e.g., antibodies). The immune system produces more protective substances from its original memory to defend itself against pathogens. In the present invention, a therapeutic vaccine including cells with the immune response is delivered into the body, which directly boosts the body's immune function, and not only increases the body's ability to resist tumor, HIV, pathogen infections, such as, viral hepatitis infections, and coronavirus or other viruses. Thus, it can be used to treat and prevent cancers, AIDS and viral hepatitis, and also has a good effect on delaying the aging process.

### BRIEF DESCRIPTION OF THE DRAWINGS

Figure 1 is a diagram showing the distribution of significant difference gene pathways of Cell 1 based on Pathway enrichment analysis in an effect example of the present invention;
Figure 2 is a diagram showing the activation interaction relationship between Cell 1 and Cell 2 signaling pathways in an effect example of the present invention; wherein circles with shaded regions represent signaling pathways remaining unchanged, circles with slashed regions represent signaling pathways activated in Cell 1, and blank circles represent signaling pathways activated in Cell 2; and
Figure 3 is a diagram showing the distribution of significant difference gene pathways of Cell 2 based on Pathway enrichment analysis in an effect example of the present invention.

### DETAILED DESCRIPTION

After conducting extensive and in-depth research and a lot of experiments and explorations, the inventor found that allogeneic mononuclear cells, cell mitogens, cytokines and immunologic adjuvants are co-cultured, so that universal immune cells (in particular, CTLs) which are safe, reliable, durable and effective, and have no rejection reaction can be prepared. In addition, the universal immune cells can provide low-cost, safe and reliable maternal(backbone) cells for other types of universal immune cell preparations, such as CAR-T and TCR-T or cellular immunotherapy of other immune cells.

In a first aspect, the invention provides a simple and convenient method for preparing universal immune cells, wherein allogeneic mononuclear cells are co-cultured with cell mitogens, cytokines and immunologic adjuvants to obtain a large number of universal immune cells. The method comprises the following steps of:
Step 1, placing the allogeneic immune cells (e.g., mononuclear cells), the cell mitogens, the cytokines and the immunologic adjuvants in a liquid cell culture medium to be co-cultured in a culture container, so that universal immune cell culture with high immunocompetence is obtained;
Step 2, isolating a cell population with immunocompetence from the culture obtained in Step 1;
wherein the cytokines are derived from any one or more of lymphokines, monokines, cytokines activating inflammation and cytokines stimulating hematopoiesis produced by lymphocytes, monocytes and other cells.

In a preferred embodiment of the present invention, the cytokines are derived from any one or more of interleukins, interferons, tumor necrosis factors, colony-stimulating factors, chemotactic cytokines and transforming growth factors; in a preferred embodiment of the present invention, the cytokines are one or more of interleukin (IL)-2, IL-6, IL-15 and interferons.

In a preferred embodiment of the present invention, the cytokines are IL-2 and/or interferons. During the co-cultivation process, the allogeneic mononuclear cells can also secrete IL-2 and other cytokines autonomously due to the stimulation of the cell mitogens, the cytokines and the immunologic adjuvants, so a lower IL-2 dependence is achieved, or even IL-2 is no longer needed. Exogenous IL-2 can also be added during the culture process, which further promotes activated T lymphocytes to amplify in large quantities.

In a preferred embodiment of the present invention, in order to obtain a culture of a cell population with immunocompetence, the concentration of the cytokines is in a range of 200,000 units/L to 5,000,000 units/L during the culture process. In a preferred embodiment of the present invention, the concentration of the cytokines is in a range of 500,000 units/L to 2,000,000 units/L.

In a preferred embodiment of the present invention, the mitogens are selected from any one or more of concanavalin, phytohaemagglutinin, pokeweed, lipopolysaccharide, and dextran.

In a preferred embodiment of the present invention, in order to obtain a culture of a cell population with immunocompetence, the concentration of the mitogens is in a range of 100,000 units/L to 10,000,000 units/L during the culture process. In a preferred embodiment of the present invention, the concentration of the mitogens is in a range of 500,000 units/L to 5,000,000 units/L.

In a preferred embodiment of the present invention, in order to obtain a culture of a cell population with immunocompetence, the concentration of the mitogens is in a range of 0.1 mg/L to 10 mg/L during the culture process. In a preferred embodiment of the present invention, the concentration of the mitogens is in a range of 0.5 mg/L to 5 mg/L.

In a preferred embodiment of the present invention, the immunologic adjuvants are selected from any one or more of biological adjuvants, inorganic adjuvants, organic adjuvants, synthetic adjuvants, oil-based adjuvants, and Freund's adjuvants; in order to obtain a culture of a cell population with immunocompetence, the concentration of the immunologic adjuvants is in a range of 0.01 mg/L to 1 mg/L during the culture process. In a preferred embodiment of the present invention, the concentration of the immunologic adjuvants is in a range of 0.1 mg/L to 0.5 mg/L.

In a preferred embodiment of the present invention, the immune cells are derived from peripheral blood or umbilical cord blood. In a preferred embodiment of the present invention, the immune cells are derived from the peripheral blood.

In a preferred embodiment of the present invention, the immune cells are allogeneic cells or autologous cells. In a preferred embodiment of the present invention, the immune cells are allogeneic cells.

In a preferred embodiment of the present invention, in order to obtain a culture of a cell population with immunocompetence, the concentration of the immune cells is in a range of 1×10³ cells/ml to 1×10¹¹ cells/ml during the culture process. In a preferred embodiment of the present invention, the concentration of the immune cells is in a range of 1×10⁵ cells/ml to 1×10⁹ cells/ml.

However, ratios of different types of immune cells (i.e., allogeneic mononuclear cells) to cell mitogens, cytokines and immunologic adjuvants may be different, and those skilled in the art can determine an appropriate ratio through experiments or by virtue of characteristics of the immune cells.

In a preferred embodiment of the present invention, the cell population with immunocompetence comprises cytotoxic T lymphocytes (CTL), tumor-infiltrating lymphocytes (TIL), cytokine-induced killer (CIK) cells, lymphokine-activated killer (LAK) cells, natural killer (NK) cells, tumor-associated macrophages (TAM), activated killer monocytes (AKM), and dendritic cells (DC).

In a preferred embodiment of the present invention, the cell population with immunocompetence is lymphocytes, for example, CTL, NK, CIK and B cells; in a more preferred embodiment, the cell population with immunocompetence is cytotoxic T lymphocytes.

In a preferred embodiment of the present invention, the cells are co-cultured for a period of 3-180 days in Step 1; in a preferred embodiment of the present invention, the cells are co-cultured for a period of 3-60 days in Step 1; in a preferred embodiment of the present invention, the cells are co-cultured for a period of 7-28 days in Step 1; in a more preferred embodiment, the cells are co-cultured for a period of 14-21 days in Step 1.

In a preferred embodiment of the present invention, the culture container is a three-dimensional cell culture container with a large volume and a high density. However, it can also be other known culture containers, including bioreactors.

In a preferred embodiment of the present invention, the method further comprises a cloning step: using the culture obtained in Step 1 or the cell population with immunocompetence obtained in Step 2 as raw materials, and performing a cloning process in a liquid cell culture medium to obtain cell lines with immunocompetence. The cloning process is performed by intermittent cyclic stimulation or continuous stimulation.

In a second aspect, the present invention provides universal immune cells prepared by the above-mentioned method. The cells comprise one or more of cytotoxic T lymphocytes (CTL), tumor-infiltrating lymphocytes (TIL), cytokine-activated killer (CLK) cells, lymphokine-activated killer (LAK) cells, natural killer (NK) cells, tumor-associated macrophages (TAM), activated killer monocytes (AKM), and dendritic cells (DC).

In a preferred embodiment of the present invention, the universal immune cells are lymphocytes, such as CTL, NK, CIK and B cells. In a more preferred embodiment, the above-mentioned universal immune cells are cytotoxic T lymphocytes.

In a third aspect, the present invention provides a use of the universal immune cells in the second aspect for the preparation of an immune cell preparation, wherein maternal(backbone) cells of the immune cell preparation are the universal immune cells.

In a preferred embodiment of the present invention, the immune cell preparation comprises CAR-T cells or TCR-T cells; the immune cell preparation can be used for adoptive cellular immunotherapy, and can be clinically used as cellular biopharmaceuticals for the treatment and prevention of various blood system disorders and various solid tumors.

In a preferred embodiment of the present invention, the above-mentioned blood system disorders comprise: 1) erythrocyte diseases, such as aplastic anemia, paroxysmal nocturnal hemoglobinuria (PNH), etc.; 2) leukocyte diseases, such as various leukemias, malignant lymphoma, malignant lympho-reticulosis, plasma cell diseases, histiocytosis, myelodysplastic syndrome, myeloproliferative diseases (polycythemia vera, essential thrombocythemia, myelofibrosis).

In a preferred embodiment of the present invention, the above-mentioned solid tumors can be selected from the group consisting of nasopharyngeal carcinoma, esophageal cancer, gastric cancer, liver cancer, breast cancer, colorectal cancer, prostate cancer, lung cancer, cervical cancer, leukemia, oral cancer, salivary gland tumor, malignant tumors of the nasal cavity and paranasal sinuses, laryngeal cancer, ear tumors, ocular tumors, thyroid tumors, mediastinal tumors, chest wall, pleural tumors, small intestine tumors, biliary tract tumors, pancreas and periampullary tumors, mesenteric and retroperitoneal tumors, kidney tumors, adrenal tumors, bladder tumors, prostate cancer, testicular tumors, penile cancer, endometrial cancer, malignant ovarian tumors, malignant trophoblastic tumors, vulvar and vaginal cancers, malignant lymphoma, multiple myeloma, soft tissue tumors, bone tumors, skin and adnexal tumors, malignant melanoma, nervous system tumors, various pediatric tumors, etc.

In a fourth aspect, the present invention provides an immune cell preparation prepared by using the universal immune cells in the second aspect as the maternal(backbone) cells.

In a fifth aspect, the present invention provides a biological drug for adoptive cellular immunotherapy, comprising a container and the immune cell preparation in the fourth aspect, placed in the container.

In a preferred embodiment of the present invention, the biological drug further comprises physiological saline, albumin and other stabilizers.

In a sixth aspect, the present invention provides a use of the immune cell preparations in the fourth aspect for the preparation of drugs for the treatment and prevention of diseases related to abnormalities in the quantity and functions of lymphocytes. The use includes but is not limited to: tumor immunity, transplantation immunity, hypersensitivity immunity, autoimmune diseases, immunoproliferative diseases, immunodeficiency diseases, infection and immunity, aging and immunity, reproduction and immunity, reproductive systems and immunity, immune blood diseases, respiratory system diseases and immunity, kidney diseases and immunity, digestive system diseases and immunity, endocrine diseases and immunity, cardiovascular system diseases and immunity, connective tissue diseases, immunodermatology, trauma and immunity, parasitosis and immunity. For example, the drugs are used for the treatment and prevention of diseases such as tumors, AIDS and viral hepatitis, coronavirus or other types of viruses, or for delaying the aging process, etc.

Wherein, the above-mentioned drugs may be a vaccine for improving the immune function of the body, or a drug for treating diseases by improving the immune function of the body. The vaccine and the drug can be prepared into any available dosage forms.

In a preferred embodiment of the present invention, routes of administration for the drugs comprise intravenous injection, intrathoracic injection, intraperitoneal injection, intraspinal injection, intradermal injection, subcutaneous injection or intratumoral injection.

The present invention will now be described more fully hereinafter with reference to particular embodiments and accompanying drawings, so as to better understand the present invention. However, the scope of the present invention is not limited to the following embodiments.

Unless otherwise stated, methods in the examples refer to conventional methods; and unless otherwise stated, reagents used herein refer to conventional commercially available reagents or reagents prepared according to the conventional methods.

### Example 1

In this example, there is provided a simple and convenient method for preparing universal immune cells, comprising the following steps:
allogeneic mononuclear cells derived from normal human peripheral blood were co-cultured with cell mitogens, cytokines and immunologic adjuvants in a liquid medium, wherein an amount of the cell mitogen, concanavalin in the medium was 100,000 units/L; an amount of the cytokine, IL-2 in the medium was 500,000 units/L; and an amount of the immunologic adjuvant (5% Tween-80) was 0.5 mL/L.

The allogeneic mononuclear cells were co-cultured with the cell mitogens, the cytokines and the immunologic adjuvants for 30 days to obtain an immune cell culture with immunocompetence, mainly cytotoxic T lymphocytes.

### Example 2

In this example, there is provided a simple and convenient method for preparing universal immune cells, comprising the following steps:
allogeneic mononuclear cells derived from allogeneic peripheral blood were co-cultured with cell mitogens, cytokines and immunologic adjuvants in a liquid medium, wherein the medium contains 0.5 mg/L cell mitogen, phytohaemagglutinin; 5,000,000 units/L cytokine, interferon; 0.1 ml/l immunologic adjuvant (5% Tween-80).

The allogeneic mononuclear cells were co-cultured with the cell mitogens, the cytokines and the immunologic adjuvants for 30 days to obtain an immune cell culture with immunocompetence, mainly cytotoxic T lymphocytes.

### Example 3

In this example, there is provided a simple and convenient method for preparing universal immune cells, comprising the following steps:
allogeneic mononuclear cells derived from normal human umbilical cord blood were co-cultured with cell mitogens, cytokines and immunologic adjuvants in a liquid medium, wherein an amount of the cell mitogen, concanavalin in the medium was 1,000,000 units/L; an amount of the cytokine, IL-2 in the medium was 1,000,000 units/L; and an amount of the immunologic adjuvant (5% Tween-80) was 0.1 mL/L.

The allogeneic mononuclear cells were co-cultured with the cell mitogens, the cytokines and the immunologic adjuvants for 45 days to obtain an immune cell culture with immunocompetence, mainly cytotoxic T lymphocytes.

### Example 4

In this example, there is provided a simple and convenient method for preparing universal immune cells, comprising the following steps:
mononuclear cells derived from allogeneic umbilical cord blood were co-cultured with cell mitogens, cytokines and immunologic adjuvants in a liquid medium, wherein the medium contains 1 mg/L of the cell mitogen, phytohaemagglutinin; 3,000,000 units/L cytokine, interferon; 0.5 ml/l immunologic adjuvant (5% Tween-80).

The allogeneic mononuclear cells were co-cultured with the cell mitogens, the cytokines and the immunologic adjuvants for 60 days to obtain an immune cell culture with immunocompetence, mainly cytotoxic T lymphocytes.

### Effect Example 1

Taking T lymphocytes as an example, a large number of activated and expanded immune cells can be obtained by using the above-mentioned method. Combination labeling, single-cell whole-gene expression sequencing and whole-gene gene expression sequencing were used. Two different groups of immune effector cells were screened and analyzed by using characteristic genes. The two different groups of immune effector cells were Cell 2 and Cell 1. Cell 2 were cells obtained by co-culturing of immune cells with cell mitogens, cytokines and immunologic adjuvants for eight days; and Cell 1 were cells obtained by co-culturing of the immune cells with the mitogens, the cytokines and the immunologic adjuvants for 26 days. The following results were the whole-gene characteristics of the immune cells found jointly by the above-mentioned two detection and analysis methods (Table 1).

**Table 1 whole-gene characteristics information of immune cells**

| Genes | log₂FoldChange | Cell Type | Molecule Description |
|---|---|---|---|
| *CFTR* | 4.91 | Cell 1 | Cystic fibrosis transmembrane conductance regulator |
| *CFH* | 4.73 | Cell 1 | Complement Factor H |
| *FGR* | 1.95 | Cell 1 | FGR proto-oncogene, Src family tyrosine kinase |
| *TMEM176A* | -1.92 | Cell 2 | Transmembrane protein 176A |
| *KLHL13* | -5.56 | Cell 2 | Kelch-like protein 13 |
| *CD38* | -6.28 | Cell 2 | CD3 molecule |

When studying differences between the two groups of samples, we took differential genes as research objects for further research. DESeq2 was used to compare the two groups to obtain the marker gene difference (Fold-change) between the samples; in this analysis, the two groups were compared to screen the differential genes under the following screening conditions: Fold change was converted into log₂FoldChange via log, genes in which log₂FoldChange>1.9 or <-1.9 were used as marker genes. It can be seen from Table 1 that differential expression characteristics of those genes were prominent.

### Effect Example 2

Taking T lymphocytes as an example, a large number of activated and expanded immune cells can be obtained by using the above method. Cell 1 were cells obtained by co-culturing of immune cells with cell mitogens, cytokines and immunologic adjuvants for 26 days; then Pathway analysis was performed.

Systematic analysis of the relationship between genes (and their encoding products), gene functions and databases of genome information helps researchers study genes and expression information as a whole network. An integrated metabolic pathway provided by KEGG comprises metabolism of carbohydrates, nucleosides, amino acids and biodegradation of organic substances. It not only provides all possible metabolic pathways, but also comprehensively annotates enzymes catalyzing all kinds of reactions in each of steps. KEGG is a powerful tool for *in vivo* metabolic analysis and metabolic network research. At present, KEGG Pathway is divided into eight categories, namely, overall network, metabolic process, genetic information transmission, environmental information transmission, intracellular biological process, biological systems, human diseases and drug development.

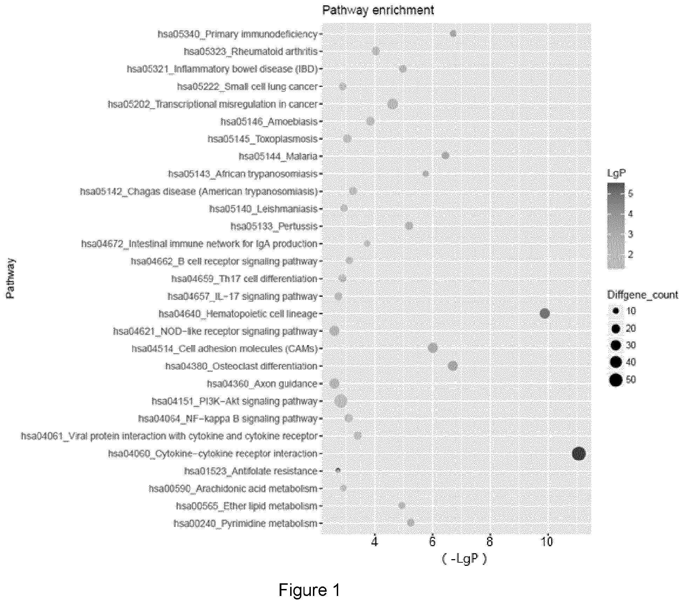

Pathway analysis is conducted based on the KEGG database. For differential genes, performing a significance analysis on pathways involved in target genes by using Fisher's exact test and Chi-square test. Pathways are screened according to *p* value<0.05, so as to obtain significant pathways. Distribution maps of significant differential gene pathways can be constructed according to a significance level (*p* value) of each differential gene pathway in differential gene pathways with upregulation and downregulation of significance in the analysis result, and the number of differential genes contained in the differential gene pathways. In the distribution maps, the ordinate is the name of the differential gene pathway, and the abscissa is a negative logarithm of the *p* value (-LgP), wherein, the larger the value, the smaller the *p* value, that is, the higher the significance level of the differential gene pathway, and the size of bubbles represents the number of the differential genes contained in the pathways. For ease of viewing for researchers, bubble maps for some (some pathways in which *p* value<0.05; if there are more pathways in which *p* value<0.05, only top 30 items having smaller *p* value will be shown) of up-regulated and down-regulated significant differential gene functions are made. Taking the up-regulated significance pathways as an example, and results displaying significance sections are shown in Figure 1.

It can be seen from Figure 1 that there are two signaling pathways enriched with marker genes. These two pathways are Hematopoietic cell lineage signaling pathway and Cytokine-cytokine receptor interaction (cytokine interaction) signaling pathway. These two signaling pathways are the main characteristic pathways for activation of Cell 1.

### Effect Example 3

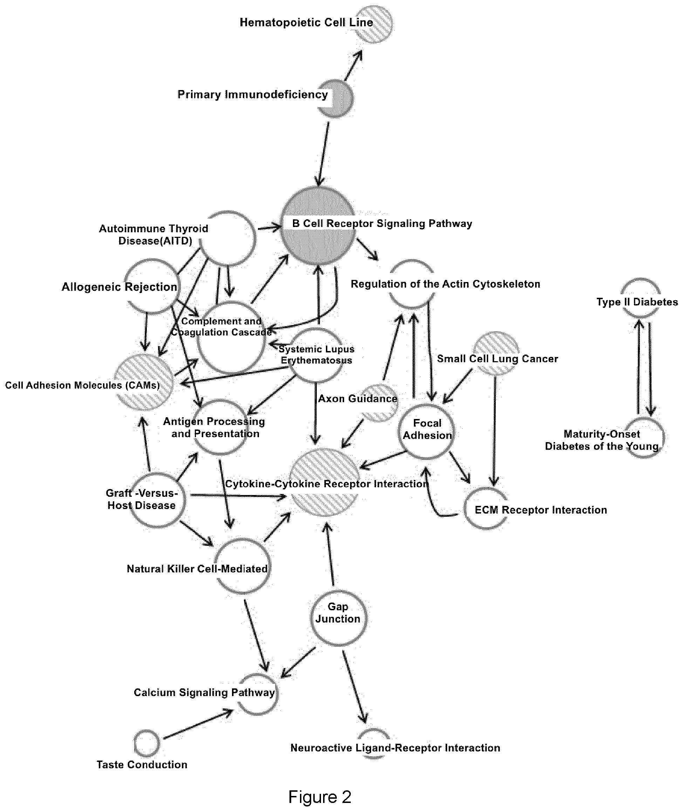

Taking T lymphocytes as an example, a large number of activated and expanded immune cells can be obtained by using the above-mentioned method. Cell 1 were cells obtained by co-culturing of immune cells with cell mitogens, cytokines and immunologic adjuvants for 26 days, Cell 2 were cells obtained by co-culturing of the immune cells with the cell mitogens, the cytokines and the immunologic adjuvants for eight days; then analyzing the activation and interaction relationship between the Cell 1 and Cell 2 signaling pathways, and results were shown in Figure 2.

In Figure 2, due to these effects, signaling pathways formed activation delivery networks, which cascaded to induce immune cell phenotypes. After analyzing all the immune signaling pathways, we found that the hematopoietic cell lineage signaling pathway, as a signature pathway of Cell 1, was stimulated by upstream signaling pathways, and it was a very important effector signaling pathway. While a cytokine-cytokine receptor interaction signaling pathway served as a core effector target for stimulation of numerous upstream signaling pathways.

### Effect Example 4

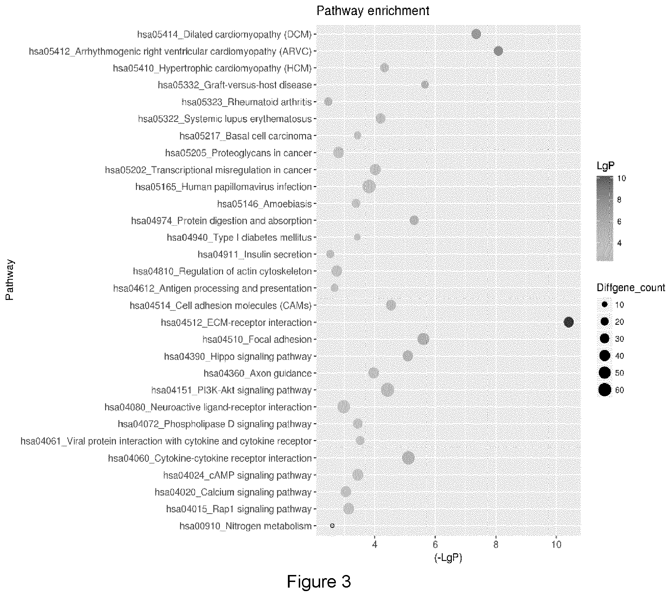

Taking T lymphocytes as an example, a large number of activated and expanded immune cells can be obtained by using the above-mentioned method. Cell 2 were cells obtained by co-culturing of immune cells with cell mitogens, cytokines and immunologic adjuvants for eight days; then Pathway analysis was performed (as shown in Figure 3). Likewise, we analyzed that the main signaling pathway activated by Cell 2 was an ECM receptor interaction signaling pathway. The enrichment degree of signature genes in this signaling pathway is the most obvious (-LgP is the largest): distribution maps of significant differential gene pathways can be constructed according to a significance level (p value) of each differential gene pathway in differential gene pathways with upregulation and downregulation of significance in the analysis result, and the number of differential genes contained in the differential gene pathways. In the distribution maps, the ordinate is name of the differential gene pathway, and the abscissa is a negative logarithm of the p value (-LgP), wherein, the larger the value, the smaller the p value, that is, the higher the significance level of the differential gene pathway. Dark or light colors represent the degree of up-regulation and down-regulation and the additive effect of genes in the signaling pathway, while the size of the bubbles represents the number of differential genes in the pathway.

Through a signaling pathway activation network, it was found that the ECM receptor interaction signaling pathway is activated by the focal adhesion signaling pathway in Cell 2 and reversely strengthens the focal adhesion signaling pathway again.

### Effect Example 5

Taking T lymphocytes as an example, a large number of activated and expanded immune cells can be obtained by using the above-mentioned method. PBMC samples were tested for cell concentration viability using Countstar^{®} Rigel fluorescence analyzer and AOPI dye method. Results were shown in Table 2.

**Table 2 Activated immune cells (Countstar^{®} Rigel fluorescence analyzer, AOPI method)**

| Samp le | Cell Viabilit y (%) | Total Cell Concentrat ion(Unit/ml ) | Viable Cell Concentr ation (Unit/ml) | Dead Cell Concentra tion (Unit/ml) | Total Cell numb er | Viable Cell Numb er | Dead Cell Numb er |
|---|---|---|---|---|---|---|---|
| 1 | 95.8% | 4.59E+06 | 4.39E+06 | 1.93E+05 | 3117 | 2986 | 131 |
| 2 | 95.68 % | 5.28E+06 | 5.05E+06 | 2.28E+05 | 3586. 5 | 3431. 5 | 155 |
| 3 | 96.54 % | 3.90E+06 | 3.77E+06 | 1.35E+05 | 2651. 5 | 2560 | 91.5 |

Immune cell subsets were analyzed by flow cytometry, and results showed that CD8⁺ T lymphocytes in samples 1, 2 and 3 were 95.34%, 96.9% and 96.2%, respectively. Those results are similar to results obtained by our previous similar methods: CD8⁺ T lymphocytes increased by more than 95%, wherein most of them are CD45RO positive cells, about 93%. All of those results indicate that the obtained immune effector cells are cytotoxic T-lymphocytes (CTLs).

### Effect Example 6

The universal immune cells prepared by the method provided by the present invention were injected into a subject to verify their safety and effectiveness.

Subject information: Liu, male, 65 years old, received a course of intravenous infusion of the allogeneic universal immune cells prepared by the above-mentioned method every three months for the past two years. Each course of treatment was divided into three venous re-transfusions. Re-transfusion was made every other day. The number of the cells involved in each of the re-transfusions was in a range of 5×10⁸ cells to 1×10¹⁰ cells.

Effects: during the whole process, he tolerated it well, and there were no clinical symptoms and signs related to rejection you can recognize in skin, a liver, an intestinal tract and other organs or systems, such as rash, jaundice and diarrhea. In addition, injection of the allogeneic universal immune cells prepared by the above-mentioned method had ten benefits: (1) age spots were reduced; (2) skin texture was improved; (3) chronic eczema disappeared; (4) white hair was reduced; (5) memory was enhanced; (6) blood lipids decreased slightly; (7) glycosylated hemoglobin returned to normal from a slight increase; (8) oral mucosal ulcers did not occur; (9) joint damage was reduced; and (10) he became more energetic.

### Effect Example 7

The universal immune cells prepared by the method provided by the present invention were injected into a subject to verify their safety and effectiveness.

Subject information: Xue, male, 56 years old, a patient with liver cancer. He received an intravenous infusion of the allogeneic universal immune cells prepared by the above-mentioned method for about four years. Initially, he received a course of intravenous infusion each month, and each course of treatment was divided into five venous re-transfusions. Two years later, he received a course of intravenous infusion every other month, and each course of treatment was still divided into five venous re-transfusions. Re-transfusion was made every other day. The number of the cells involved in each of the re-transfusions was in a range of 5×10⁸ cells to 1×10⁹ cells.

Effects: during the whole process, he tolerated it well, except for the occasional short-term fever, and no other adverse reactions were observed. No abnormality was observed in repeated blood tests, liver function tests and renal function tests. Various physical examinations, including imageological examination, indicated that the tumor was in complete remission.

### Effect Example 8

The universal immune cells prepared by the method provided by the present invention were injected into a subject to verify their safety and effectiveness.

Subject information: Wang, female, 75 years old, a patient with colon cancer. She received an intravenous infusion of the allogeneic universal immune cells prepared by the above-mentioned method for about ten years, specifically, she received an intravenous infusion of the allogeneic universal immune cells once a month. The number of the cells involved in each of the venous re-transfusions was 5×10⁹ cells.

Effects: during the whole process, she tolerated it well, and no other adverse reactions were observed. There were no clinical symptoms and signs related to rejection you can recognize in skin, a liver, an intestinal tract and other organs or systems, such as rash, jaundice and diarrhea. No abnormality was observed in repeated blood tests, liver function tests and renal function tests. The patient did not have any clinical symptoms and signs, and abnormalities were not observed in all blood tests and tumor markers. Colonoscopy re-examination revealed no abnormalities. The patient complained of markedly reduced cold symptoms.

### Effect Example 9

The universal immune cells prepared by the method provided by the present invention were injected into a subject to verify their safety and effectiveness.

Subject information: Li, male, 70 years old, a patient with malignant melanoma. Over the past four years, he was injected with the allogeneic universal immune cells prepared by the above-mentioned method via various routes, for example, venous re-transfusion, intraperitoneal infusion and subcutaneous (intratumoral) injection. Initially, he received a course of treatment every two weeks. However, he later received a course of treatment every month, every three months, or every six months. Each course of treatment was divided into four venous re-transfusions. Re-transfusion was made every other day. The number of the cells involved in each of the re-transfusions was in a range of 5×10⁸ cells to 1×10⁹ cells.

Effects: during the whole process, he tolerated it well, except for pain during subcutaneous injection, and no other adverse reactions were observed. No abnormality was observed in repeated blood tests, liver function tests and renal function tests. In addition, there were no clinical symptoms and signs related to rejection you can recognize in skin, a liver, an intestinal tract and other organs or systems, such as rash, jaundice and diarrhea. The patient had advanced malignant melanoma. However, it recurred and metastasized after surgery, and chemotherapy was ineffective. Of note, the tumor was well controlled by applying a single treatment using the universal immune cells prepared by the above-mentioned method.

Particular embodiments are described fully hereinbefore. However, those embodiments are given by way of example only and are not intended to be limiting of the invention. It should be understood by those skilled in the art that equivalent modifications and substitutes to the application fall within the scope of the invention. Thus, various changes and modifications are all within the scope of the invention without departing from the scope or spirit of the invention.

## Claims

1. A method for preparing universal immune cells, comprising the steps of:
Step 1, placing allogeneic immune cells, cell mitogens, cytokines and immunologic adjuvants in a liquid cell culture medium to be co-cultured in a culture container, so that universal immune cell culture is obtained;
Step 2, isolating a cell population from the culture obtained in Step 1;
wherein the cytokines are derived from any one or more of lymphokines, monokines, cytokines activating inflammation and cytokines stimulating hematopoiesis produced by lymphocytes, monocytes and other cells.

2. The method of claim 1, wherein the cytokines are derived from any one or more of interleukins, interferons, tumor necrosis factors, colony-stimulating factors, chemotactic cytokines and transforming growth factors.

3. The method of claim 1, wherein the cytokines are one or more of interleukin (IL)-2, IL-6, IL-15 and interferons.

4. The method of claim 1, wherein the concentration of the cytokines is in a range of 200,000 units/L to 5,000,000 units/L.

5. The method of claim 1, wherein the concentration of the cytokines is in a range of 500,000 units/L to 2,000,000 units/L.

6. The method of claim 1, wherein the mitogens are selected from any one or more of concanavalin, phytohaemagglutinin, pokeweed, lipopolysaccharide, and dextran.

7. The method of claim 6, wherein the concentration of the mitogens is in a range of 100,000 units/L to 10,000,000 units/L.

8. The method of claim 6, wherein the concentration of the mitogens is in a range of 0.1 mg/L to 10 mg/L.

9. The method of claim 1, wherein the immunologic adjuvants are selected from any one or more of biological adjuvants, inorganic adjuvants, organic adjuvants, synthetic adjuvants, oil-based adjuvants, and Freund's adjuvants, and the concentration of the immunologic adjuvants is in a range of 0.01 mg/L to 1 mg/L.

10. The method of claim 1, wherein the immune cells are allogeneic cells or autologous cells, and are derived from peripheral blood or umbilical cord blood.

11. The method of claim 1, wherein the concentration of the immune cells is in a range of 1×10³ cells/ml to 1×10¹¹ cells/ml.

12. The method of claim 1, wherein the cell population comprises cytotoxic T lymphocytes, tumor-infiltrating lymphocytes, cytokine-induced killer cells, lymphokine-activated killer cells, natural killer cells, tumor-associated macrophages, activated killer monocytes, and dendritic cells.

13. The method of claim 1, wherein the cells are co-cultured for a period of 3-180 days in Step 1.

14. The method of claim 1, wherein the culture container is a three-dimensional cell culture container with a large volume and a high density.

15. The method of claim 1, further comprising a cloning step:
using the culture obtained in Step 1 or the cell population obtained in Step 2 as raw material, and performing a cloning process in a liquid cell culture medium to obtain cell lines;
the cloning process is performed by intermittent cyclic stimulation or continuous stimulation.

16. A universal immune cell prepared by the method of any one of claims 1-15, comprising one or more of cytotoxic T lymphocytes, tumor-infiltrating lymphocytes, cytokine-activated killer cells, lymphokine-activated killer cells, natural killer cells, tumor-associated macrophages, activated killer monocytes, and dendritic cells.

17. A use of the universal immune cell of claim 16 for the preparation of an immune cell preparation, wherein maternal cells of the immune cell preparation are the universal immune cells.

18. The use of claim 17, wherein the immune cell preparation comprises CAR-T cells or TCR-T cells.

19. An immune cell preparation prepared by using the universal immune cells of claim 16 as the maternal cells.

20. A biological drug for adoptive cellular immunotherapy, comprising a container and the immune cell preparation of claim 11 placed in the container.

21. The biological drug of claim 20, further comprising: physiological saline, albumin and other stabilizers.

22. A use of the immune cell preparation of claim 19 for the preparation of drugs for the treatment and prevention of diseases related to abnormalities in quantity and functions of lymphocytes.

23. The use of claim 22, wherein routes of administration comprise intravenous injection, intrathoracic injection, intraperitoneal injection, intraspinal injection, intradermal injection, subcutaneous injection or intratumoral injection.
